# EUROPEAN PATENT SPECIFICATION

(11) **EP 2 241 339 B1**
(45) Date of publication and mention of the grant of the patent: **14.01.2015**
(21) Application number: 09707693.9
(22) Date of filing: 05.02.2009
(51) Int. Cl.: A61L 27/00, A61K 6/033

(54) **CALCIUM PHOSPHATE COMPOSITION AND PROCESS FOR PRODUCTION THEREOF**
CALCIUMPHOSPHATZUSAMMENSETZUNG UND HERSTELLUNGSVERFAHREN DAFÜR
COMPOSITION DE PHOSPHATE DE CALCIUM ET SON PROCÉDÉ DE FABRICATION

(30) Priority: 07.02.2008 JP 2008027827
(43) Date of publication of application: 20.10.2010
(73) Proprietor: Kuraray Noritake Dental Inc., Kurashiki-shi Okayama 710-0801 (JP)
(72) Inventor: HASHIMOTO, Tadashi, Kurashiki-shi Okayama 710-0801 (JP); OKADA, Koichi, Chiyoda-ku, Tokyo 100-0004 (JP)
(74) Representative: Müller-Boré & Partner Patentanwälte PartG mbB
(86) International application number: PCT/JP2009/051949
(87) International publication number: WO 2009/099136

(56) References cited:
- EP-A2- 1 541 321
- EP-A2- 1 541 321
- WO-A1-01/41821
- WO-A1-2007/040253
- WO-A1-2007/083601
- JP-A- 3 141 956
- JP-A- 7 048 219
- JP-A- 2007 190 169
- JP-A- 2007 190 226
- JP-A- 2007 191 318
- JP-A- 2007 191 420
- JP-A- 2007 197 329
- US-A1- 2009 258 966
- US-A1- 2010 236 449
- DATABASE WPI Week 199436 Thomson Scientific, London, GB; AN 1994-290821 XP002694915, & JP 6 219918 A (LION CORP) 9 August 1994 (1994-08-09) -& DATABASE EPODOC [Online] EUROPEAN PATENT OFFICE, THE HAGUE, NL; XP002694916, -& JP 6 219918 A (LION CORP; MITSUI TOATSU CHEMICALS) 9 August 1994 (1994-08-09)
- DATABASE WPI Week 200761 Thomson Scientific, London, GB; AN 2007-644376 XP002694917, & JP 2007 191420 A (KURARE MEDICAL KK) 2 August 2007 (2007-08-02) -& DATABASE EPODOC [Online] EUROPEAN PATENT OFFICE, THE HAGUE, NL; XP002694918, -& JP 2007 191420 A (KURARAY MEDICAL INC) 2 August 2007 (2007-08-02)
- DATABASE WPI Week 200769 Thomson Scientific, London, GB; AN 2007-731323 XP002694919, & JP 2007 191318 A (KURARE MEDICAL KK) 2 August 2007 (2007-08-02) -& DATABASE EPODOC [Online] EUROPEAN PATENT OFFICE, THE HAGUE, NL; XP002694920, & JP 2007 191318 A (KURARAY MEDICAL INC) 2 August 2007 (2007-08-02)
- CHUA B. ET AL.: 'Developing hydroxyapatite reinforced polysulphone for medical applications' PROCESSING AND FABRICATION OF ADVANCED MATERIALS vol. 8, 2000, pages 185 - 191, XP008138704
- MORITA M. ET AL.: 'Calcium Phosphate Hone Paste eno Fun'ekihi no Eikyo ni Taisuru Rikigakuteki, Soshikigakuteki Kento' ANNUAL REPORT OF ASSOCIATION OF DOCTORS WORKING IN OSAKA CITY March 2004, pages 219 - 223, XP008142149
- INAGE I ET AL: 'Dental hardenable compsn. having sufficient fluidity on filling tooth root - contains tri:calcium alpha-phosphate, X=ray contrast agent, fluorine cpd., unsatd. carboxylic acid or sulphonic acid polymer and water' WPI / THOMSON, AN 1994-290821 vol. 1994, no. 36, 09 August 1994, XP002694915 -& DATABASE EPODOC EUROPEAN PATENT OFFICE, THE HAGUE, NL; XP002694916, -& JP H06 219 918 A (LION CORP; MITSUI TOATSU CHEMICALS) 09 August 1994

## Description

### TECHNICAL FIELD

The present invention relates to calcium phosphate compositions. It particularly relates to a calcium phosphate composition suitable for a medical material, and a process for production thereof.

### BACKGROUND ART

Hydroxyapatite (Ca₁₀(PO₄)₆(OH)₂), which is obtained by sintering a calcium phosphate composition, has a composition close to inorganic components of bones, teeth and the like and has a bioactivity, which is a property of bonding directly to bones. Therefore, its use as a material for repairing bone defects or bone voids has been reported. However, although a material comprising such hydroxyapatite excels in biocompatibility, it might be difficult to apply it to a site with a complicated shape in respect of moldability.

On the other hand, it is known that among calcium phosphate compositions, a cement type of composition, that is, a calcium phosphate composition having setting property is converted gradually to living body-absorbable hydroxyapatite in a living body or in an oral cavity and moreover it can integrate with a biological hard tissue while maintaining its form. Such a calcium phosphate composition is reported to not only excel in biocompatibility but also be able to be applicable to a site with a complicated shape because it has moldability.

For example, JP 2007-190226 A (patent document 1) discloses a calcium phosphate composition comprising tetracalcium phosphate particles having an average particle diameter of 5 to 30 µm and calcium hydrogen phosphate particles having an average particle diameter of 0.1 to 5 µm, wherein the tetracalcium phosphate particles comprise 2 to 20% by weight of particles having a particle diameter of 1.5 µm or less. This reports that a calcium phosphate composition which has a setting time within an appropriate range and has good sealing ability can be provided. However, a calcium phosphate composition paste filled in a desired site is not necessarily good in sealing ability and therefore improvement has been desired.

JP 2007-99674 A (patent document 2) (US-A-2009258966) discloses a setting resin composition particularly suitable for dental use comprising a polymerizable monomer containing an acidic group, a polymerizable monomer containing a basic group, a specific reactive monomer, and a calcium filler comprising tetracalcium phosphate (TTCP) and dicalcium phosphate (DCP) in combination, and there are provided a carboxyl group and its acid anhydride group, a phosphoric acid group, a thiophosphoric acid group, a sulfonic group, and a sulfinic acid group as examples of the acidic group of the polymerizable monomer containing an acidic group. According to this, it is reported to be able to provide a dental composition which develops excellent adhering effect. Moreover, it is considered that a composition for dental use which does not generate minute leakage has been desired. However, a composition for dental use obtained in such a manner does not necessarily have good sealing ability and its improvement has been desired.

Patent document 1: JP 2007-190226 A
Patent document 2: JP 2007-99674 A
JP6219918 discloses hardenable dental composition, comprising tricalcium alpha phosphate and sulfonic acid salt polymer. Example 5 discloses compositions which further comprise dibasic calcium phosphate hydrate.

### DISCLOSURE OF THE INVENTION

### PROBLEMS TO BE SOLVED BY THE INVENTION

The present invention was made in order to solve the above-described problems. An object thereof is to provide a calcium phosphate composition that has a time between the addition of a liquid agent to the calcium phosphate composition and the completion of setting in the use at a clinical site, i.e., a setting time which is within an appropriate range, and that has high mechanical strength and that has good marginal sealing ability. Further, an object thereof is also to provide a process for producing such a calcium phosphate composition and to provide suitable application of such a calcium phosphate composition.

### MEANS FOR SOLVING THE PROBLEMS

The above-mentioned problems are solved by providing a calcium phosphate composition powder and/or a calcium phosphate composition paste by the process as defined in claims 1, 8 and 9.

It is preferable that the polysulfonic acid salt is a polystyrenesulfonic acid salt and/or a polyvinylsulfonic acid salt. Moreover, it is preferable that an alkali metal salt of phosphoric acid (C) is contained, and it is preferable that the alkali metal salt of phosphoric acid (C) is disodium hydrogen phosphate and/or sodium dihydrogen phosphate. The calcium phosphate particles (A) consist of tetracalcium phosphate particles (A1) and acidic calcium phosphate particles (A2), and it is preferable that the acidic calcium phosphate particles (A2) are anhydrous calcium hydrogen phosphate particles, and it is preferable that a blending ratio (A1/A2) of the tetracalcium phosphate particles (A1) to the acidic calcium phosphate particles (A2) are from 40/60 to 60/40 in molar ratio. It is preferable that the composition contain acidic calcium phosphate composite particles which contains 1 to 30 parts by weight of inorganic particles (D) other than (A1) and (A2) based on 100 parts by weight of the acidic calcium phosphate particles (A2) and in which the acidic calcium phosphate particles (A2) are covered with the inorganic particles (D), and it is preferable that the inorganic particles (D) have an average particle diameter of from 0.002 to 2 µm. Moreover, it is preferable that the inorganic particles (D) are particles of silica or a metal oxide, and it is also preferable that the acidic calcium phosphate composite has an average particle diameter of from 0.1 to 7 µm. Moreover, the embodiment that the calcium phosphate composition is a powder is also a preferable embodiment.

Moreover, the above-mentioned problems are solved by providing a kit for medical use which comprises the compositions produced by the process according to claims 1, 8 and 9.

Moreover, the above-mentioned problems are solved by providing a process for producing a calcium phosphate composition powder comprising calcium phosphate particles (A)
and a polysulfonic acid salt (B), wherein the calcium phosphate composition powder contains 0.5 to 20 parts by weight of the polysulfonic acid salt (B) based on 100 parts by weight of the calcium phosphate particles (A), wherein the calcium phosphate particles (A) consist of tetracalcium phosphate particles (A1) and acidic calcium phosphate particles (A2), and the tetracalcium phosphate particles (A1), the acidic calcium phosphate particles (A2) and the polysulfonic acid salt (B) are mixed in a state of a powder, and it is preferable that the acidic calcium phosphate particles (A2) and inorganic particles (D) other than (A1) and (A2) are mechanochemically hybridized and then mixed with the tetracalcium phosphate particles (A1) and the polysulfonic acid salt (B) in a state of a powder. It is preferable that at least one device selected from among a jet mill, a pestle and mortar machine, a ball mill, a bead mill, a planetary mill, a hybridizer, a mechanofusion machine, or a kneading extruder is used in the hybridization, and in particular to use a vibrating ball mill is a preferable embodiment.

Moreover, the above-mentioned problems are also solved by providing a process for producing a calcium phosphate composition paste comprising calcium phosphate particles (A) and a polysulfonic acid salt (B), wherein a liquid comprising water as a main component is added to a powder of a calcium phosphate composition comprising 0.5 to 20 parts by weight of the polysulfonic acid salt (B) based on 100 parts by weight of the calcium phosphate particles (A), followed by kneading, wherein the calcium phosphate particles (A) consist of tetracalcium phosphate particles (A1) and acidic calcium phosphate particles (A2), and the tetracalcium phosphate particles (A1), the acidic calcium phosphate particles (A2) and the polysulfonic acid salt (B) are mixed in a state of a powder.

Moreover, the above-mentioned problems are also solved by providing a process for producing a calcium phosphate composition paste comprising calcium phosphate particles (A) and a polysulfonic acid salt (B), wherein an aqueous solution containing 0.5 to 20 parts by weight of the polysulfonic acid salt (B) based on 100 parts by weight of the calcium phosphate particles (A) is added to a powder comprising the calcium phosphate particles (A), followed by kneading wherein the calcium phosphate particles (A) consist of tetracalcium phosphate particles (A1) and acidic calcium phosphate particles (A2). The calcium phosphate composition is, in a preferred embodiment, a composition for medical use and particularly it is suitable as a bone cement or a filling material for dental use.

### EFFECT OF THE INVENTION

The calcium phosphate composition of the present invention has a time between the addition of a liquid agent to the calcium phosphate composition and the completion of setting in the use at a clinical site and the like, i.e., a setting time which is within an appropriate range, and it is high in mechanical strength and good in marginal sealing ability. Therefore, it is suitable for materials for medical use and it
is suitable for, especially, a bone cement and a filling material for dental use.

### BRIEF DESCRIPTION OF THE DRAWINGS

[Fig. 1] An SEM photograph of the anhydrous calcium hydrogen phosphate composite particles obtained in Example 15.
[Fig. 2] An SEM photograph of a large particle in the mixture of anhydrous calcium hydrogen phosphate particles (A2) and silica particles obtained in Reference Example.
[Fig. 3] An SEM photograph of a small particle A in the mixture of anhydrous calcium hydrogen phosphate particles (A2) and silica particles obtained in Reference Example.
[Fig. 4] An SEM photograph of a small particle B in the mixture of anhydrous calcium hydrogen phosphate particles (A2) and silica particles obtained in Reference Example.

### BEST MODE FOR CARRYING OUT THE INVENTION

The calcium phosphate composition produced by the process of the present invention contains calcium phosphate particles (A) and a polysulfonic acid salt (B).

A calcium phosphate composition containing calcium phosphate particles (A) will be set when being kneaded in the presence of water. It has become clear that at this time by further containing a polysulfonic acid salt (B) in addition to the calcium phosphate particles (A), the sealing ability of a site filled with a calcium phosphate composition paste having a setting time within an appropriate range and maintaining strong compression strength is improved remarkably. While the reason for this is not necessarily clear, the following mechanism is presumed.

That is, it is conceivable that when a calcium phosphate composition paste containing a polysulfonic acid salt (B) is prepared, the polysulfonic acid salt (B) functions as a surfactant, so that calcium phosphate composition particles become good in dispersibility and are packed efficiently, and it is expected that as a result of the foregoing, the marginal sealing ability is improved. Although causes are not necessarily clear, it is conceivable that the presence of the polysulfonic acid salt (B) makes hydroxyapatite form between calcium phosphate composition particles efficiently and the hydroxyapatite closes holes formed when a calcium phosphate composition filled in a desired site is set, so that marginal sealing ability is improved. In practice, a tendency that a hole formed in a set product of a calcium phosphate composition is made smaller by the addition of a polysulfonic acid salt (B) is observed, and the polysulfonic acid salt (B) is considered to influence the deposition form and size of hydroxyapatite.

The calcium phosphate particles (A) to be used for the present invention consist of tetracalcium phosphate particles (A1) and acidic calcium phosphate particles (A2).

It is preferable that the average particle diameter of the calcium phosphate particles (A) to be used for the present invention is from 0.5 to 30 µm. When the average particle diameter is smaller than 0.5 µm, the viscosity of a paste to be obtained by mixing with a liquid agent may become excessively high and the strength of a set product may lower. The average particle diameter of the calcium phosphate particles (A) is more preferably 2 µm or more, and even more preferably 5 µm or more. On the other hand, when the average particle diameter is greater than 30 µm, a paste to be obtained by mixing with a liquid agent may have unsatisfactory paste properties, for example, it may not show a sufficiently high viscosity. In use as a root canal filler or the like for dental use, when it is injected to a narrow implantation site with a syringe, the tip of a nozzle may be clogged therewith. The average particle diameter of the calcium phosphate particles (A) is more preferably 25 µm or less, and even more preferably 20 µm or less. Here, the average particle diameter of the calcium phosphate particles (A) to be used for the present invention is a value calculated by measuring all particles which can constitute the calcium phosphate particles (A), namely tetracalcium phosphate particles (A1) and acidic calcium phosphate particles (A2), by using a laser diffraction type particle size distribution analyzer.

The calcium phosphate composition of the present invention contains 0.5 to 20 parts by weight of the polysulfonic acid salt (B) based on 100 parts by weight of the calcium phosphate particles (A). When the content of the polysulfonic acid salt (B) is less than 0.5 part by weight, sealing ability may deteriorate, and it is preferably 2 parts by weight or more, and more preferably 5 parts by weight or more. On the other hand, when the content of the polysulfonic acid salt (B) exceeds 20 parts by weight, a setting time becomes long and sealing ability and compression strength lower, and the viscosity at the time of preparing a calcium phosphate composition paste becomes high and, as a result, it may become difficult to knead the paste; therefore, the content is preferably 18 parts by weight or less, and more preferably 15 parts by weight or less.

Polysulfonic acid salts are used from the viewpoint that the marginal sealing ability of a site filled with a calcium phosphate composition paste becomes good. In the present invention, while the cation of the polysulfonic acid salt (B) is not particularly restricted and may be lithium, sodium, potassium, ammonium, and the like, sodium or potassium is preferably used.

The term "polysulfonic acid salt" represents a product resulting from polymerization of a sulfonic acid salt monomer or a product resulting from introduction of a sulfo group to a polymer by a sulfonation reaction. The polysulfonic acid salt to be used for the present invention is not particularly restricted, and examples thereof include a polystyrenesulfonic acid salt, a polynaphthalenesulfonic acid salt, a polynaphthylmethanesulfonic acid, a polyvinylsulfonic acid salt, and the like. Among them, a polystyrenesulfonic acid salt and/or a polyvinylsulfonic acid salt is used preferably, and both a polystyrenesulfonic acid salt and a polyvinylsulfonic acid salt are used more preferably from the viewpoint that the marginal sealing ability of a site filled with a calcium phosphate composition paste becomes good.

As to the molecular weight of the polysulfonic acid salt to be used for the present invention, a polysulfonic acid salt with an arbitrary molecular weight between oligomers with a molecular weight of less than 100 and crosslinked polymers can be used. In particular, it is preferable that the molecular weight is from 1000 to 10 million Da (Dalton). When the molecular weight is less than 1000 Da, neither an effect of improvement in the compression strength of a set product of a calcium phosphate composition nor an effect of the reduction of a setting time of the composition may be expected and sealing ability may deteriorate, and therefore the molecular weight is more preferably 5000 Da or more and even more preferably 10, 000 Da or more. On the other hand, when the molecular weight exceeds 10 million Da, the viscosity at the time of preparing a calcium phosphate composition paste may become high, so that it may become difficult to perform kneading, and therefore the molecular weight is more preferably 8 million Da or less, and even more preferably 6 million Da or less.

It is preferable that the calcium phosphate composition of the present invention further contains an alkali metal salt of phosphoric acid (C). This can make the setting time shorter, and therefore the operativity is improved and the sealing ability can be improved. The alkali metal salt of phosphoric acid (C) to be used is not particularly restricted, and examples thereof include disodium hydrogen phosphate, dipotassium hydrogen phosphate, lithium dihydrogen phosphate, sodium dihydrogen phosphate, potassium dihydrogen phosphate,
trisodium phosphate, tripotassium phosphate, and so on, among which one salt or two or more salts are used. Among them, from the viewpoint of safety and ease with which high purity raw materials can be obtained, it is preferable that the alkali metal salt of phosphoric acid (C) is disodium hydrogen phosphate and/or sodium dihydrogen phosphate.

The calcium phosphate particles (A) to be used for the present invention consist of tetracalcium phosphate particles (A1) and acidic calcium phosphate particles (A2).
When a calcium phosphate composition containing tetracalcium phosphate particles (A1) and acidic calcium phosphate particles (A2) is kneaded in the presence of water, it forms thermodynamically stable hydroxyapatite to be set. By inclusion of 0.5 to 20 parts by weight of a polysulfonic acid salt (B) in a calcium phosphate composition consisting of tetracalcium phosphate particles (A1) and acidic calcium phosphate particles (A2), a calcium phosphate composition which has a setting time within an appropriate range and is high in mechanical strength and good in sealing ability is obtained.

A method for producing the tetracalcium phosphate [Ca₄(PO₄)₂O] particles (A1) to be used for the present invention is not particularly restricted. Commercially available tetracalcium phosphate particles may be used as it is, or alternatively, they may be used after appropriate regulation of their particle size by grinding. As a grinding method, a method which is the same as the grinding method of acidic calcium phosphate particles (A2) described below can be used.

It is preferable that the tetracalcium phosphate particles (A1) have an average particle diameter of from 5 to 30 µm. When the average particle diameter is less than 5 µm, the tetracalcium phosphate particles (A1) dissolve excessively, so that the pH of the aqueous solution becomes so high that hydroxyapatite does not deposit smoothly and, as a result, the mechanical strength of a set product may lower. The average particle diameter is more preferably 10 µm or more. On the other hand, when the average particle diameter is greater than 30 µm, a paste to be obtained by mixing with a liquid agent may have undesirable paste properties, for example, it may not exhibit a sufficient viscosity or it may exhibit an increased rougher feeling. In use as a root canal filler or the like for dental use, when it is injected to a narrow implantation site with a syringe, the tip of a nozzle may be clogged therewith. The average particle diameter is more preferably 25 µm or less. Here, the average particle diameter of the tetracalcium phosphate particles (A1) to be used for the present invention is calculated through measurement using a laser diffraction type particle size distribution analyzer.

While the acidic calcium phosphate particles (A2) to be used for the present invention may be either such anhydrous particles as anhydrous calcium hydrogen phosphate [CaHPO₄] particles, monocalcium phosphate anhydrous [Ca(H₂PO₄)₂] particles, and calcium dihydrogen pyrophosphate [CaH₂P₂O₇], or such hydrous particles as dicalcium phosphate dihydrate [CaHP0₄·2H₂O] particles and monocalcium phosphate monohydrate [Ca(H₂PO₄)₂·H₂O] particles, anhydrous particles are used preferably, and in particular anhydrous calcium hydrogen phosphate [CaHPO₄] particles are used more preferably. In the present invention, the molar number of phosphate radials was defined as the molar number of the acidic calcium phosphate particles (A2). It is preferable that the average particle diameter of the acidic calcium phosphate particles (A2) is from 0.1 to 5 µm. When the average particle diameter is less than 0.1 µm, the viscosity of a paste to be obtained by mixing with a liquid agent may become excessively high, and it is more preferably 0.5 µm or more. On the other hand, when the average particle diameter exceeds 5 µm, the acidic calcium phosphate particles (A2) become less soluble in a liquid agent and, as a result, excessive dissolution of tetracalcium phosphate particles (A1) occurs. Then, it causes increase in pH of the aqueous solution, which will inhibit hydroxyapatite from depositing smoothly, so that the mechanical strength of a set product may lower. The average particle diameter is more preferably 2 µm or less. The average particle diameter of the acidic calcium phosphate particles (A2) is calculated in the same manner as the average particle diameter of the tetracalcium phosphate particles (A1).

A method for producing the acidic calcium phosphate particles (A2) having such an average particle diameter is not particularly restricted. While commercial products may be used if available, it is often preferable to further grind a commercially available product. In such a case, a grinding machine, such as a ball mill, a pestle and mortar machine and a jet mill, can be used. Acidic calcium phosphate particles (A2) can also be obtained by grinding a raw material powder of acidic calcium phosphate together with such a liquid medium as alcohol by the use of a pestle and mortar machine, a ball mill, or the like to prepare a slurry, and drying the obtained slurry. As the grinding machine in this process, a ball mill is preferably used. As the material of its pot and balls, alumina or zirconia is preferably used.

As described above, by adjusting the average particle diameter of the tetracalcium phosphate particles (A1) to be larger that the average particle diameter of the acidic calcium phosphate particles (A2), the solubilities of both of the materials are balanced and the pH of an aqueous solution is maintained almost neutral, and it thereby is possible to make the formation of hydroxyapatite crystals smooth and to increase the mechanical strength of a set product. Specifically, it is more preferable to adjust the average particle diameter of (A1) to be not less than twice, even more preferably not less than four times, and particularly preferably not less than seven times the average particle diameter of (A2). On the other hand, it is more preferable to adjust the average particle diameter of (A1) to be not more than 35 times, even more preferably not more than 30 times, and particularly preferably not more than 25 times the average particle diameter of (A2).

While the blending ratio (A1/A2) of the tetracalcium phosphate particles (A1) to the acidic calcium phosphate particles (A2) is not particularly restricted, it is preferable for the particles to be used in a blending ratio within the range of from 40/60 to 60/40 in molar ratio. Thereby, a calcium phosphate composition from which a set product having high mechanical strength is formed can be obtained. The blending ratio (A1/A2) is more preferably from 45/55 to 55/45, and most preferably is substantially 50/50.

The embodiment that the composition is a calcium phosphate composition containing acidic calcium phosphate composite particles which further contains inorganic particles (D) other than (A1) and (A2) and in which the acidic calcium phosphate particles (A2) are covered with the inorganic particles (D) is a preferable embodiment. As described above, it has become clear that by the inclusion of the acidic calcium phosphate composite particles which contains the inorganic particles (D) other than (A1) and (A2) and in which the acidic calcium phosphate particles (A2) are covered with the inorganic particles (D), the sealing ability of a site filled with a calcium phosphate composition paste is improved remarkably. While the reason for this is not necessarily clear, the following mechanism is presumed.

In the preparation of the calcium phosphate composition of the present invention, by mechanochemically hybridizing acidic calcium phosphate particles (A2) and inorganic particles (D), acidic calcium phosphate composite particles in which the surface of the acidic calcium phosphate particles (A2) is covered with the inorganic particles (D) are obtained. For example, when silica particles are used as the inorganic particles (D), acidic calcium phosphate composite particles in which the surface of the acidic calcium phosphate particles (A2) is covered with the silica particles are obtained. Subsequently, by mixing the acidic calcium phosphate composite particles and tetracalcium phosphate particles (A1), the calcium phosphate composition of the present invention is obtained. When a calcium phosphate composition paste is prepared by mixing a calcium phosphate composition containing acidic calcium phosphate composite particles and a liquid containing water as a main component, it seems that tetracalcium phosphate particles (A1) and acidic calcium phosphate particles (A2) are dissolved and at the same time hydroxyapatite are formed efficiently starting at polar groups of the inorganic particles (D) located on the surface of the acidic calcium phosphate composite particles. For example, it seems that when silica particles are used as the inorganic particles (D), hydroxyapatite is formed efficiently starting at silanol groups of the silica particles. In particular, by performing a treatment of mechanochemically grinding by using a ball mill or the like as in the present invention, polar groups of the inorganic particles (D) are expected to increase and hydroxyapatite seems to be formed efficiently. At this time, it is conceivable that marginal sealing ability is improved by closing with hydroxyapatite a hole formed when a calcium phosphate composition filled in a desired site is set. Moreover, it is considered that since acidic calcium phosphate particles (A2) are smaller in average particle diameter in comparison to tetracalcium phosphate particles (A1), gaps between the tetracalcium phosphate particles (A1) are packed efficiently, and therefore it is conceivable that marginal sealing ability is improved.

The kind of the inorganic particles (D) to be used for the present invention is not particularly restricted, and it is preferably silica or at least one member selected from among metal oxides. Specific examples of the metal oxide include titania, alumina, zirconia, cerium oxide (ceria), hafnium oxide (hafnia), yttrium oxide (yttria), beryllium oxide (beryllia), niobium oxide (niobia), lanthanum oxide, bismuth oxide, tin oxide, zinc oxide, iron oxide, molybdenum oxide, nickel oxide, ytterbium oxide, samarium oxide, europium oxide, praseodymium oxide, magnesium oxide, and neodymium oxide. Among them, titania, zirconia, or alumina is used preferably as a metal oxide. The inorganic particles (D) to be used for the present invention are preferably at least one member selected from among silica, titania, zirconia, or alumina, more preferably at least one member selected from among silica, titania, or zirconia, and particularly preferably silica.

It is preferable that the average particle diameter of the inorganic particles (D) to be used for the present invention is from 0.002 to 2 µm. When the average particle diameter is less than 0.002 µm, the viscosity of the calcium phosphate composition becomes so high that the handleability may deteriorate, and the average particle diameter is more preferably 0.003 µm or more, and even more preferably 0.005 µm or more. On the other hand, when the average particle diameter exceeds 2 µm, the setting time of a calcium phosphate composition paste becomes long and marginal sealing ability also may become poorer; therefore it is more preferably 1 µm or less, even more preferably 0.5 µm or less, and particularly preferably 0.2 µm or less. The average particle diameter of the inorganic particles (D) is calculated by observing primary particles dispersed in an epoxy resin by using a transmission electron microscope.

The calcium phosphate composition of the present invention preferably contains 1 to 30 parts by weight of the inorganic particles (D) based on 100 parts by weight of the acidic calcium phosphate particles (A2). When the content of the inorganic particles (D) is less than 1 part by weight, sealing ability may deteriorate, and it is more preferably 2 parts by weight or more, and even more preferably 5 parts by weight or more. On the other hand, when the content of the inorganic particles (D) exceeds 30 parts by weight, the setting time becomes long and sealing ability may deteriorate, and it is preferably 20 parts by weight or less, and more preferably 15 parts by weight or less.

In the preparation of the calcium phosphate composition of the present invention, by mechanochemically hybridizing acidic calcium phosphate particles (A2) and inorganic particles (D), acidic calcium phosphate composite particles in which the acidic calcium phosphate particles (A2) are covered with the inorganic particles (D) are obtained. Here, the acidic calcium phosphate composite particles refer to composite particles in which the surface of acidic calcium phosphate particles (A2) is covered almost uniformly with inorganic particles (D). That is, the acidic calcium phosphate composite particles refer to composite particles in which the surface of acidic calcium phosphate particles (A2) are covered almost uniformly with inorganic particles (D) so that the existing ratio of the inorganic particles (D) on the surface of the acidic calcium phosphate composite particles may become almost uniform. According to the result of the elemental analysis by the use of an energy dispersive X-ray analyzer (EDX) in Examples described later in which silica particles are used as the inorganic particles (D), the existing ratio of Si element at an arbitrary site on the surface of an acidic calcium phosphate composite particle was almost the same regardless of location. When anhydrous calcium hydrogen phosphate particles, monocalcium phosphate anhydrous particles, dicalcium phosphate dihydrate particles, monocalcium phosphate monohydrate particles, or calcium dihydrogen pyrophosphate particles are used as acidic calcium phosphate particles (A2), anhydrous calcium hydrogen phosphate composite particles, monocalcium phosphate anhydrous composite particles, dicalcium phosphate dihydrate composite particles, monocalcium phosphate monohydrate composite particles, or calcium dihydrogen pyrophosphate composite particles are obtained, respectively.

It is preferable that the average particle diameter of the acidic calcium phosphate composite particles to be used for the present invention is from 0.1 to 7 µm. When the average particle diameter is less than 0.1 µm, the viscosity of a paste to be obtained by mixing with a liquid agent may become excessively high, and it is more preferably 0.5 µm or more. On the other hand, when the average particle diameter exceeds 7 µm, because of the decrease in surface area and covering with the inorganic particles (D) thickly, the acidic calcium phosphate particles (A2) may become less soluble in a liquid agent. Moreover, excessive dissolution of tetracalcium phosphate particles (A1) occurs, so that the pH of a setting reaction system becomes high and, as a result, hydroxyapatite is inhibited from depositing smoothly and thus the mechanical strength of a set product may lower. Therefore, the average particle diameter is more preferably 3 µm or less.

The calcium phosphate composition of the present invention may contain components other than tetracalcium phosphate particles (A1), acidic calcium phosphate particles (A2), a polysulfonic acid salt (B), an alkali metal salt of phosphoric acid (C), and at least one member of inorganic particles (D) selected from among silica or a metal oxide within the range in which the effect of the present invention is not adversely affected. For example, a thickener may be blended according to need. This is for improving the moldability or uniform filling property of a calcium phosphate composition paste. The thickener may be, for example, one or two or more species selected from among carboxymethylcellulose, sodium carboxymethylcellulose, hydroxypropylcellulose, hydroxypropylmethylcellulose, polyvinyl alcohol, polyethylene glycol, polyacrylic acid, polyglutamic acid, polyglutamic acid salts, polyaspartic acid, polyaspartic acid salts, starch other than cellulose, alginic acid, hyaluronic acid, polysaccharides such as pectin, chitin and chitosan, acidic polysaccharide esters such as propylene glycol alginate, and polymers such as proteins, e.g. collagen, gelatin and their derivatives. From aspects of solubility in water and viscosity preferred is at least one species selected from sodium carboxymethylcellulose, hydroxypropylcellulose, hydroxypropylmethylcellulose, alginic acid, chitosan, polyglutamic acid and polyglutamic acid salts. The thickener may be blended to a calcium phosphate powder, or may be blended to a liquid agent, or may be incorporated to a paste under kneading.

An X-ray contrast medium may also be contained according to need. This is because the operation of filling a calcium phosphate composition paste can be monitored or change of the paste after its filling can be traced. Examples of the X-ray contrast medium include one or two or more agents selected from among barium sulfate, bismuth subnitrate, bismuth oxide, bismuth subcarbonate, ytterbium fluoride, iodoform, barium apatite, barium titanate, and so on. The X-ray contrast medium may be incorporated to a calcium phosphate powder, or may be incorporated to a liquid agent, or may be incorporated to a paste under kneading.

Moreover, any pharmacologically acceptable agents may be incorporated. For example, disinfectants, anticancer agents, antibiotics, antibacterial agents, blood circulation improvers such as actosin and PEG1, growth factors such as bFGF, PDGF and BMP, cells which promotes hard tissue formation, such as osteoblasts, odontoblasts, and anaplastic bone marrow derived stem cells may be incorporated.

While the calcium phosphate composition of the present invention contains calcium phosphate particles (A) and a polysulfonic acid salt (B), the calcium phosphate composition of the present invention is preferably a powder. At this time, a calcium phosphate composition powder is produced by mixing the calcium phosphate particles (A) and the polysulfonic acid salt (B) in a state of a powder. Tetracalcium phosphate particles (A1), acidic calcium phosphate particles (A2), and a polysulfonic acid salt (B) are mixed in a state of a powder. The mixing method is not particularly restricted; for example, a jet mill, a pestle and mortar machine, a ball mill, a bead mill, a planetary mill, a hybridizer, a mechanofusion machine, a kneading extruder, a high speed rotation mill, and so on can be used, a pestle and mortar machine, a ball mill, a planetary mill, or a high speed rotation mill is preferably used, and a high speed rotation mill is more preferably used. It is also possible to mix in the presence of a water-free liquid medium such as alcohol. Moreover, mixing a polysulfonic acid salt (B) separately with tetracalcium phosphate particles (A1) and acidic calcium phosphate particles (A2), followed by mixing the resulting mixtures can also be performed as a preferable embodiment.

Furthermore, the calcium phosphate composition of the present invention can be obtained also by mechanochemically hybridizing acidic calcium phosphate particles (A2) with inorganic particles (D) and then mixing them with tetracalcium phosphate particles (A1) and a polysulfonic acid salt (B). At this time, by mechanochemically hybridizing acidic calcium phosphate particles (A2) and inorganic particles (D), acidic calcium phosphate composite particles in which the surface of the acidic calcium phosphate particles (A2) is covered with the inorganic particles (D) are obtained. This fact can provide a calcium phosphate composition with good marginal sealing ability.

The method of the mechanochemical hybridization is not particularly restricted, and it is preferable to use at least one device selected from among a jet mill, a pestle and mortar machine, a ball mill, a bead mill, a planetary mill, a hybridizer, a mechanofusion machine, or a kneading extruder, and it is more preferable to use a vibrating ball mill. Although a specific device to be used for the hybridization is not restricted, an example of the hybridizer is a hybridization system manufactured by Nara Machinery Co., Ltd., an example of the mechanofusion machine is a circulation type mechanofusion system AMS manufactured by Hosokawa Micron Group, and an example of the kneading extruder is a KEX extruder manufactured by Kurimoto, Ltd.

By mixing the thus-obtained acidic calcium phosphate composite particles with tetracalcium phosphate particles (A1) and a polysulfonic acid salt (B), a calcium phosphate composition with good marginal sealing ability is obtained. A mixing method to be used for mixing the calcium phosphate particles (A) and the polysulfonic acid salt (B) in a state of a powder is adopted.

When the calcium phosphate composition of the present invention comprising calcium phosphate particles (A) and a polysulfonic acid salt (B) is a powder composition, in its use in a medical site, it can be used by being kneaded with a liquid containing water as a main component, i.e., a liquid agent to form a calcium phosphate composition paste, and being filled in or applied to a desired site. The liquid containing water as a main component may be pure water or alternatively may be an aqueous solution or an aqueous dispersion containing other components.

As a component to be blended with water, an alkali metal salt of phosphoric acid (C) is preferably contained. As the alkali metal salt of phosphoric acid (C), the aforementioned salts can be used. Moreover, pH buffers, such as ammonium phosphates, N,N-bis(2-hydroxyethyl)-2-aminoethanesulfonic acid, and N-tris(hydroxymethyl)methyl-2-aminoethanesulfonic acid, fluoride salts, such as sodium fluoride, potassium fluoride, and ammonium fluoride, water-soluble polyhydric alcohols, such as glycerin and propylene glycol can also be used.

In the present invention, a calcium phosphate composition paste can also be obtained by mixing an aqueous solution containing a polysulfonic acid salt (B) not to a calcium phosphate composition containing calcium phosphate particles (A) and a sulfonic acid salt (B) but to a powder of a calcium phosphate composition containing calcium phosphate particles (A). That is, a calcium phosphate composition paste is produced by adding an aqueous solution containing 0.5 to 20 parts by weight of the polysulfonic acid salt (B) based on 100 parts by weight of the calcium phosphate particles (A) to a powder containing the calcium phosphate particles (A), followed by kneading, wherein the calcium phosphate particles (A) consist of tetracalcium phosphate particles (A1) and acidic calcium phosphate particles (A2).

The sulfonic acid salt (B) is not necessarily needed to be dissolved in water uniformly, and it may have formed a micelle in water or alternatively may be in the form of a water-insoluble microparticle emulsion or slurry.

While the mass ratio of a calcium phosphate composition to a liquid agent (calcium phosphate composition/liquid agent) in the preparation of the calcium phosphate composition paste is not particularly limited, it is preferably from 1 to 6, and more preferably from 3 to 5. The calcium phosphate composition and the liquid agent are kneaded well so that they can be mixed uniformly, and then they are filled in or applied to a desired site promptly.

The calcium phosphate composition of the present invention can be used by being filled in or applied to a desired site in the form of a calcium phosphate composition paste as described above. At this time, the calcium phosphate composition paste is usually prepared at a medical site. Therefore, a kit for medical use which comprises a powder comprising calcium phosphate particles (A) and a polysulfonic acid salt (B) and a liquid comprising water as a main component is an embodiment of the present invention. Moreover, a kit for medical use which comprises a powder comprising calcium phosphate particles (A) and an aqueous solution comprising a polysulfonic acid salt (B) is also an embodiment of the present invention.

The calcium phosphate composition of the present invention is used suitably for various medical applications. For example, it is preferably used as a bone cement for adhering or fixing. When the calcium phosphate composition of the present invention is used for this application, it can be filled to all parts with a complicated form because of excellent filling property of a paste, and therefore it is suitable for a material for restoring hard tissue, such as teeth and bones. Moreover, when an affected part on the surface of which a dental tubule exists, such as a cut root canal, is filled with a calcium phosphate composition as a filling material for dental use, good sealing ability is exhibited due to efficient formation of hydroxyapatite crystals occurring in a hole, and therefore the composition is used preferably as a root canal filler or a root canal restorative material. Furthermore, it is excellent in biocompatibility because the paste itself is converted to hydroxyapatite within a short period of time in a living body or an oral cavity, resulting in integration with a biological hard tissue.

### EXAMPLES

The present invention is illustrated below more concretely with reference to Examples. In the Examples, regarding an average particle diameter of calcium phosphate particles (A), tetracalcium phosphate particles (A1), acidic calcium phosphate particles (A2), and acidic calcium phosphate composite particles, measurement was conducted using a laser diffraction type particle size distribution analyzer ("SALD-2100" manufactured by Shimadzu Corporation), and a median diameter calculated from the result of the measurement was defined as the average particle diameter.

### Example 1

### (1) Preparation of calcium phosphate composition

Tetracalcium phosphate particle (A1) to be used for the experiments were prepared as follows. A cake-like equimolar mixture obtained by adding commercially available anhydrous calcium hydrogen phosphate particles (Product No. 1430, made by J. T. Baker Chemical Co.) and calcium carbonate particles (Product No. 1288, made by J. T. Baker Chemical Co.) in equimolar amount to water, followed by stirring for one hour, filtering and drying was heated in a calcination machine (Model 51333, manufactured by Lindberg, Watertown, WI) at 1500°C for 24 hours, and then a TTCP (tetracalcium phosphate) mass was prepared by cooling the mixture to room temperature in a desiccator. Subsequently, the resulting TTCP mass was crushed roughly with a mortar, and then an impalpable powder and TTCP mass were removed by sieving, so that the particle size was regulated into a range of 0.5 to 3 mm. Thus, rough tetracalcium phosphate particles were obtained. Moreover, tetracalcium phosphate particles (A1) (21. 2 µm in average particle diameter) to be used for the Examples were obtained by adding 100 g of the rough tetracalcium phosphate particles and 300 g of zirconia balls with a diameter of 20 mm into a 400-ml grinding pot made of alumina ("Type A-3 HD pot mill" manufactured by Nikkato Corporation), and followed by grinding at a rotation speed of 200 rpm for 2 hours and 30 minutes.

As one example of the acidic calcium phosphate particles (A2), anhydrous calcium hydrogen phosphate particles (A2) to be used for this experiment (1.1 µm in average particle diameter) were obtained by subjecting a slurry resulting from addition of 50 g of commercially available anhydrous calcium hydrogen phosphate particles (Product No. 1430, made by J. T. Baker Chemical Co., 10. 3 µm in average particle diameter), 120 g of 95% ethanol ("Ethanol (95)" made by Wako Pure Chemical Industries, Ltd.) and 240 g of zirconia balls having a diameter of 10 mm into a 400-ml grinding pot made of alumina ("Type A-3 HD pot mill" manufactured by Nikkato Corporation) and subsequent wet grinding at a rotation speed of 120 rpm for 24 hours, to evaporate ethanol with a rotary evaporator, followed by drying at 60°C for 6 hours and additional vacuum drying at 60°C for 24 hours.

A calcium phosphate composition was obtained by mixing 145.8 g of the above-mentioned tetracalcium phosphate particles (A1) and 54.2 g of anhydrous calcium hydrogen phosphate particles (A2) by using a high speed rotation mill ("SM-1" manufactured by As One Corporation). In this process, there was substantially no change in the average particle diameter between before and after the mixing with the tetracalcium phosphate particles (A1) and the calcium hydrogen phosphate particles (A2). The average particle diameter of the calcium phosphate particles (A) obtained by mixing the above-mentioned tetracalcium phosphate particles (A1) and the anhydrous calcium hydrogen phosphate particles (A2) in the above-mentioned proportions was 16.8 µm.

### (2) Measurement of compressive strength

A liquid agent was prepared by adding distilled water to 10 g of a PSS-1 powder obtained by freeze-drying an aqueous PSS-1 (sodium polystyrene sulfonate) solution ("PS-100" made by TOSOH ORGANIC CHEMICAL CO., LTD., molecular weight: about 1,000,000 Da, solid content: 20%), 51.2 g of an aqueous PVSA (poly (sodium vinylsulfonate)) solution (made by Polysciences Inc., solid content: 25%), and 3.2 g of Na₂HPO₄ (disodium hydrogen phosphate) so that the whole amount might become 100 g. A calcium phosphate composition paste was prepared by precisely weighing 1 g of the calcium phosphate composition obtained above, and adding thereto 0.25 g of the liquid agent prepared above (containing 2.5 parts by weight of PSS-1, 3.2 parts by weight of PVSA, and 0.8 part by weight of Na₂HPO₄, respectively, based on 100 parts by weight of (A1) and (A2) in total), followed by kneading (the mixed weight ratio (powder/liquid) at this time was 4). A calcium phosphate composition paste was molded (n = 9) by placing a separable mold made stainless steel having a diameter of 6 mm and a depth of 3 mm on a smooth glass plate, filling the paste therein with care being taken not to incorporate gas, and compressing it from above with a smooth glass plate. Then, after an incubation continued for 4 hours in an environment of 37°C and a 100% relative humidity, a cylindrical set product of the calcium phosphate composition was taken out from the mold and was immersed and further held for 20 hours in 150 ml of distilled water of 37°C. Then, the compressive strength of the set product of the calcium phosphate composition was measured (n = 9) by applying a load at a rate of 1 mm/min to the top face of the cylindrical set product by using a dynamic strength analyzer ("AG-1 100kN" manufactured by Shimadzu Corporation) in accordance with the method of Chow et al. (L. C. Chow, S. Hirayama, S. Takagi, and E. Parry, J. Biomed. Mater. Res. (Appl. Biomater.) 53: 511-517, 2000). The compressive strength of the set product of the calcium phosphate composition obtained from Example 1 was 55.2 ± 2.0 MPa.

### (3) Measurement of setting time

A setting time of the calcium phosphate composition obtained in (1) above was measured by the method in accordance with ISO 6876 (Dental root canal sealing materials). A calcium phosphate composition paste was prepared by precisely weighing 1 g of the calcium phosphate composition, and adding thereto a liquid agent prepared in the same manner as (2) above, followed by kneading (the mixed weight ratio (powder/liquid) at this time was 4). The calcium phosphate composition paste was filled in a ring-shaped mold made of stainless steel having a cavity with an internal diameter of 10 mm and a height of 2 mm placed on a glass plate, in a cabinet conditioned at 37°C and a 98% relative humidity, and the top of the paste was made smooth with a spatula. Subsequently, an indicator of 100 g in weight with a flat end face of 2 mm in diameter was lowered slowly perpendicularly to a vertical face of the calcium phosphate composition paste at every 10 seconds from 180 seconds after the completion of the kneading, and this operation was repeated until a mark of the needle tip disappeared, and thus a time between the kneading and the disappearance of the needle tip mark was measured (n = 5). The setting time of the calcium phosphate composition obtained from Example 1 was 6 minutes and 28 seconds ± 12 seconds.

### (4) Measurement of marginal leakage distance

A calcium phosphate composition paste was prepared by precisely weighing 1 g of the calcium phosphate composition obtained above, and adding thereto a liquid agent prepared in the same manner as (2) above, followed by kneading (the mixed weight ratio (powder/liquid) at this time was 4), and it was used for measurement of a marginal leakage distance. The measurement of a marginal leakage distance was performed in accordance with a method of testing minute leakage of ISO/TS11405 (Second Edition, Dental materials - Testing of adhesion to tooth structure). After removing the root and the pulp of a single-root-canal bovine tooth, the root part was sealed with a dental composite resin ("AP-X" made by Kuraray Medical Inc.). Subsequently, the center of a buccal surface was polished with #80 sandpaper and then with #2000 sandpaper, so that a flat surface of enamel was produced. A cavity having about 3 mm in diameter and about 2.5 mm in depth was formed in the surface of the enamel by using a high speed handpiece for dental use. The aforementioned paste was filled into the cavity and then was set by being held under conditions of 37°C and a 98% relative humidity for 4 hour. Next, the sample was stored in distilled water of 37°C for 24 hours and then it was immersed in a 0.2% basic fuchsin solution adjusted to pH 7.2 and stored at 25°C for 24 hours. Subsequently, the central part of the cavity was cut with a water-cooled diamond blade ("Isomet" manufactured by Buehler Ltd.), and an enlarged image of a cross section was observed by using a microscope (manufactured by KEYENCE CORPORATION). The distance where a dye had entered deepest in an interface between dentine and the material was defined as a marginal leakage distance (n = 5). The marginal leakage distance of the calcium phosphate composition obtained from Example 1 was 0.21 ± 0.06 mm. The results obtained are summarized in Table 1.

### Example 2

A calcium phosphate composition paste was prepared and evaluated in the same manner as Example 1 except for separating a 0.25-gram portion (containing 5 parts by weight of a PSS-1 powder and 4.8 parts by weight of PVSA based on 100 parts by weight of (A1) and (A2) in total) from a solution prepared by adding distilled water to 10 g of a PSS-1 powder and 76.8 g of an aqueous PVSA solution to dissolve them and adjusting the whole amount to 100 g, and using it as a liquid agent instead of using a mixed solution of the PSS-1 powder, the aqueous PVSA solution, and Na₂HPO₄ in Example 1. The results obtained are summarized in Table 1.

### Example 3

A calcium phosphate composition paste was prepared and evaluated in the same manner as Example 1 except for separating a 0.25-gram portion (containing 5 parts by weight of a PSS-1 powder and 0.8 part by weight of Na₂HPO₄ based on 100 parts by weight of (A1) and (A2) in total) from a solution prepared by adding distilled water to 10 g of a PSS-1 powder and 3.2 g of Na₂HPO₄ to dissolve them and adjusting the whole amount to 100 g, and using it as a liquid agent instead of using a mixed solution of the PSS-1 powder, the aqueous PVSA solution, and Na₂HPO₄ in Example 1. The results obtained are summarized in Table 1.

### Example 4

A calcium phosphate composition paste was prepared and evaluated in the same manner as Example 1 except for separating a 0.25-gram portion (containing 5 parts by weight of a PSS-1 powder, based on 100 parts by weight of (A1) and (A2) in total) from a solution prepared by adding distilled water to 10 g of a PSS-1 powder only to dissolve it and adjusting the whole amount to 100 g, and using it as a liquid agent instead of using a mixed solution of the PSS-1 powder, the aqueous PVSA solution, and Na₂HPO₄ in Example 1. The results obtained are summarized in Table 1.

### Example 5

A calcium phosphate composition was obtained by adding 1.6 g of a PSS-1 powder (0.8 part by weight based on 100 parts by weight of (A1) and (A2) in total) in the form of solid to 145.8 g of the above-mentioned tetracalcium phosphate particles (A1) and 54.2 g of anhydrous calcium hydrogen phosphate particles (A2), followed by mixing by using a high speed rotation mill ("SM-1" manufactured by As One Corporation). A calcium phosphate composition paste was prepared by adding 0.25 g of water as a liquid agent to 1 g of the obtained calcium phosphate composition, followed by kneading, and then evaluation was done in the same manner as Example 1. The results obtained are summarized in Table 1.

### Examples 6 and 7

Calcium phosphate compositions were prepared in the same manner as Example 5 except for changing the content of a PSS-1 powder to 5 parts by weight (Example 6) and to 18 parts by weight (Example 7), respectively, based on 100 parts by weight of (A1) and (A2) in total, and then calcium phosphate composition pastes were prepared by adding water and kneading and were evaluated. The results obtained are summarized in Table 1.

### Examples 8 to 11

In each Example, a calcium phosphate composition paste was prepared and evaluated in the same manner as Example 1 except for using the sodium polystyrene sulfonate powder provided below instead of the PSS-1 powder. The results obtained are summarized in Table 1.
Example 8: Powder obtained by freeze drying an aqueous PSS-2 solution ("PS-50" made by TOSOH ORGANIC CHEMICAL CO. , LTD., about 500,000 Da in molecular weight)
Example 9: Powder obtained by freeze drying an aqueous PSS-3 solution ("PS-5" made by TOSOH ORGANIC CHEMICAL CO., LTD., about 50,000 Da in molecular weight)
Example 10: PSS-4 powder (made by Polymer Standard Service, about 2,260,000 Da in molecular weight)
Example 11: PSS-5 powder (made by Polymer Standard Service, about 5,640,000 Da in molecular weight)

### Examples 12 and 13

Calcium phosphate composition pastes were prepared and evaluated in the same manner as Example 1 except for separating a 0.25-gram portion (containing 5 parts by weight of a PSS-1 powder or PVSA (poly (sodium vinylsulfonate)) based on 100 parts by weight of (A1) and (A2) in total) from a solution prepared by adding distilled water to 20 g of DBSS (sodium dodecylbenzenesulfonate), made by Wako pure Chemical Industries, Ltd., to dissolve it and adjusting the whole amount to 100 g (Example 12 comparative example) or from a solution prepared by adding distilled water to 80 g of an aqueous PVSA solution (made by Polysciences, Inc., solid content: 25%) to dissolve it and adjusting the whole amount to 100 g (Example 13), and using them as a liquid agent instead of using a mixed solution of the PSS-1 powder, the aqueous PVSA solution, and Na₂HPO₄ in Example 1. The results obtained are summarized in Table 1.

### Example 14

A calcium phosphate composition was obtained and evaluated in the same manner as Example 1 except for changing the molar ratio (A1/A2) of the tetracalcium phosphate particles (A1) to the anhydrous calcium hydrogen phosphate particles (A2) to 0.7 in Example 1. The results obtained are summarized in Table 1.

### Example 15

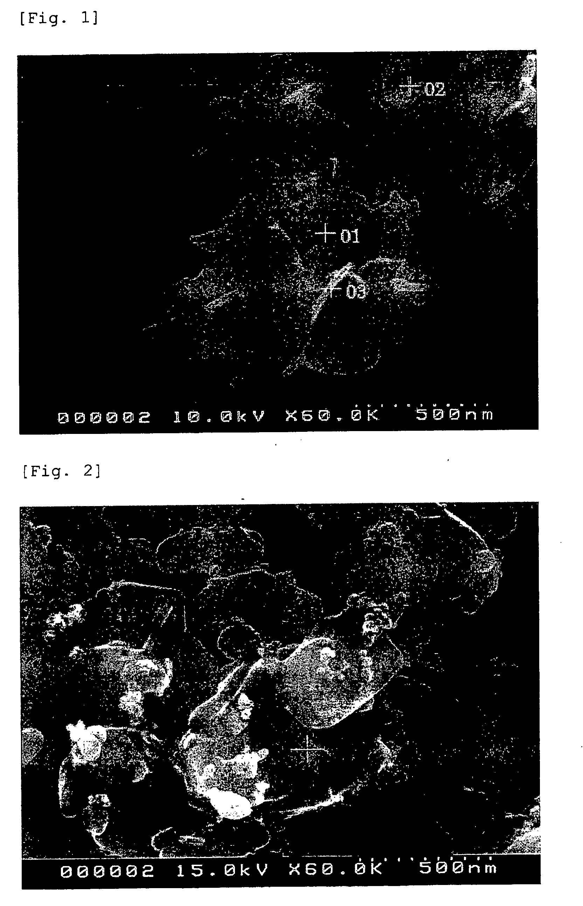

Anhydrous calcium hydrogen phosphate composite particles covered with silica particles were obtained by charging 200 g of the anhydrous calcium hydrogen phosphate particles (A2) obtained in Example 1, 20 g of silica particles ("AEROSIL 130" made by Degussa Co., average particle diameter: 0.016 µm), and 2000 g of zirconia balls having a diameter of 10 mm into a grinding pot made of alumina ("Type HD-B-104 pot mill" manufactured by Nikkato Corporation) and performing dry grinding for 20 hours by using a vibrating ball mill ("NLM" manufactured by Chuo Kakoki Shoji Inc.). The average particle diameter of the obtained anhydrous calcium hydrogen phosphate composite particles was 1.2 µm. Here, the obtained anhydrous calcium hydrogen phosphate composite particles were observed by using a field emission electron microscope (FE-SEM, "Model S-4200") manufactured by Hitachi Ltd., and anhydrous calcium hydrogen phosphate composite particles in which plate-like particle had been joined randomly to the surface of spherical particles as shown in the SEM photograph of Fig. 1 measured at a high magnification were observed. For a measuring point (+01) of the spherical particle and measuring points (flat face: +02, side face: +03) of the plate-like particle, elemental analysis was done by using an energy dispersive X-ray analyzer (EDX, "Model EMAX-5770") manufactured by HORIBA, Ltd. Results of the obtained SEM/EDX semiquantitative analysis values are summerized in Table 2.

A calcium phosphate composition was obtained by mixing 59.6 g of the anhydrous calcium hydrogen phosphate composite particles and 145.8 g of the above-mentioned tetracalcium phosphate particles (A1) by using a high speed rotation mill ("SM-1" manufactured by As One Corporation). At this time, the content of the silica particles in the calcium phosphate composition was 10 parts by weight based on 100 parts by weight of the anhydrous calcium hydrogen phosphate particles (A2). Moreover, there was substantially no change in average particle diameter between before and after the mixing with the tetracalcium phosphate particles (A1), the anhydrous calcium hydrogen phosphate particles (A2) and the silica particles.

A calcium phosphate composition paste was prepared by precisely weighing 1 g of the calcium phosphate composition obtained above, and adding thereto a liquid agent prepared in the same manner as Example 1, followed by kneading, and then the paste was evaluated. The results obtained are summarized in Table 1.

### Comparative Example 1

A calcium phosphate composition paste was prepared and evaluated in the same manner as Example 1 except for separating a 0.25-gram portion (containing 0.8 part by weight based on 100 parts by weight of (A1) and (A2) in total) from a solution prepared by dissolving 3.2 g of Na₂HPO₄ (disodium hydrogen phosphate) in 100 g of distilled water, and using it as a liquid agent instead of using a mixed solution of the PSS-1 powder, the aqueous PVSA solution, and Na₂HPO₄ in Example 1. The results obtained are summarized in Table 1.

### Comparative Example 2

A calcium phosphate composition paste was prepared and evaluated in the same manner as Example 1 except for separating a 0.25-gram portion (containing 5 parts by weight of PVA (polyvinyl alcohol) and 0.8 part by weight of Na₂HPO₄ based on 100 parts by weight of (A1) and (A2) in total) from a solution prepared by adding distilled water to 20 g of PVA (polyvinyl alcohol) made by Kuraray Co., Ltd. and 3.2 g of Na₂HPO₄ to dissolve them and adjusting the whole amount to 100 g, and using it as a liquid agent instead of using a mixed solution of the PSS-1 powder, the aqueous PVSA solution, and Na₂HPO₄ in Example 1. The results obtained are summarized in Table 1.

### Comparative Example 3

A calcium phosphate composition paste was prepared and evaluated in the same manner as Example 1 except for separating a 0.25-gram portion (containing 5 parts by weight of AGR (sodium alginate) and 0.8 part by weight of Na₂HPO₄ based on 100 parts by weight of (A1) and (A2) in total) from a solution prepared by adding distilled water to 20 g of AGR made by Wako Pure Chemical Industries, Ltd. and 3.2 g of Na₂HPO₄ to dissolve them and adjusting the whole amount to 100 g, and using it as a liquid agent instead of using a mixed solution of the PSS-1 powder, the aqueous PVSA solution, and Na₂HPO₄ in Example 1. The results obtained are summarized in Table 1.

### Comparative Example 4

A calcium phosphate composition paste was prepared and evaluated in the same manner as Example 1 except for separating a 0.25-gram portion (containing 5 parts by weight of ACR (poly(sodium acrylate)) based on 100 parts by weight of (A1) and (A2) in total) from a solution prepared by adding distilled water to 20 g of ACR only to dissolve it and adjusting the whole amount to 100 g, and using it as a liquid agent instead of using a mixed solution of the PSS-1 powder, the aqueous PVSA solution, and Na₂HPO₄ in Example 1. The results obtained are summarized in Table 1. As to an aqueous ACR solution, there was used a product obtained by neutralizing a polyacrylic acid made by Wako Pure Chemical Industries, Ltd. with a 1M aqueous NaOH solution, followed by freeze-drying.

### Comparative Example 5

A calcium phosphate composition paste was prepared and evaluated in the same manner as Example 4 except for using 0.3 part in the amount of a PSS-1 powder by weight based on 100 parts by weight of (A1) and (A2) in total in Example 4. The results obtained are summarized in Table 1.

### Comparative Example 6

A calcium phosphate composition paste was prepared and evaluated in the same manner as Example 5 except for using 22 parts in the amount of a PSS-1 powder by weight based on 100 parts by weight of (A1) and (A2) in total in Example 5. The results obtained are summarized in Table 1.

### Referential Example

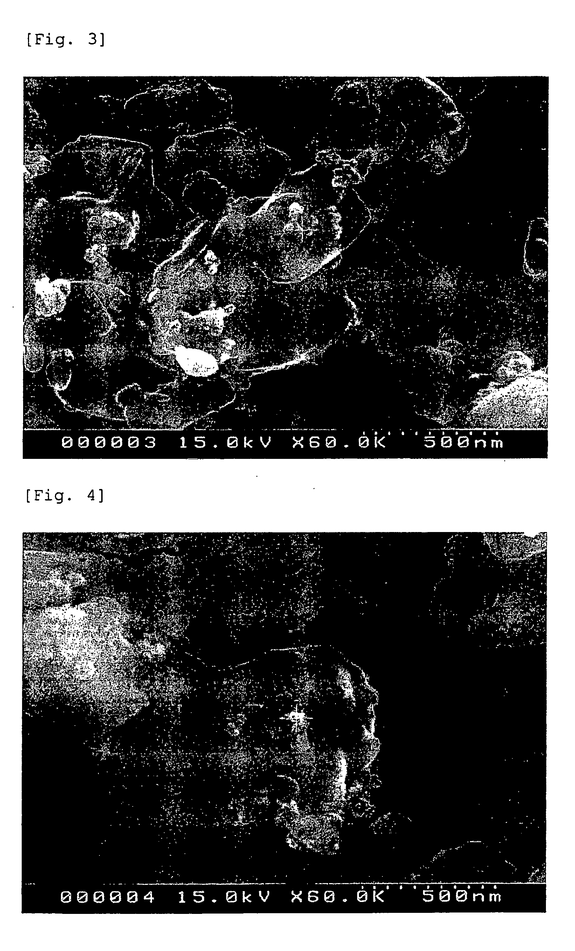

A mixture of anhydrous calcium hydrogen phosphate particles (A2) and silica particles was obtained by grinding anhydrous calcium hydrogen phosphate particles (A2) and silica particles for 20 hours by using a rotary blade grinder (high speed rotation mill) ("SM-1" manufactured by As One Corporation) instead of using a vibration ball mill in Example 15. Here, the mixture was observed by using a field emission electron microscope (FE-SEM, "Model S-4200") manufactured by Hitachi Ltd. in the same manner as Example 15, so that large particles (average particle diameter: about 1 µm) and small particles (average particle diameter: about 10 nm) were observed as shown by the SEM photographs of Figs. 2 to 4. For measuring points (+) of a large particle and a small particle (A and B) of Figs. 2 to 4, elemental analysis was done by using an energy dispersive X-ray analyzer (EDX, "Model EMAX-5770") manufactured by HORIBA, Ltd. Results of SEM/EDX semiquantitative analysis values excluding C (carbon) are summerized in Table 3 and results of SEM/EDX semiquantitative analysis values including C (carbon) are summerized in Table 4.

**[Table 1]**

| | Mixing method (*1) | Kind (*2) and blended amount (part by weight) | Blended amount of sulfonic acid salt (B) (part by weight) | Inorganic particle (D) | | | TTCP/DCPA (*3) (mol/mol) | Compressive strength (MPa) | Setting time (ISO6876) | Marginal leakage distance (mm) |
|---|---|---|---|---|---|---|---|---|---|---|
| | | | | Kind | Particle diameter (mm) | Blended amount (parts by weight) | | | | |
| Example 1 | L | PSS-1 (2.5) | 7.5 | **-** | - | - | 1 | 5.5.2±2.0 | 6 min 28 sec± 12 sec | 0.21 ±0.06 |
| | | PVSA (3.2) | | | | | | | | |
| | | Na₂HPO₄ (0.8) | | | | | | | | |
| Example 2 | L | PSS-1 (5) | 9.8 | - | - | - | 1 | 50.2±1.6 | 19 min 22 sec+23 sec | 0.25±0.09 |
| | | PVSA (4.8) | | | | | | | | |
| Example 3 | L | PSS-1 (5) | 5 | - | - | - | 1 | 55.5±2.1 | 6 min 42 sec±19 sec | 0.27±0.12 |
| | | Na₂HPO₃ (0.8) | | | | | | 55.5±.2.1 | 6 min 42 sec±19 sec | 0.27±0.12 |
| Example 4 | L | PSS-1 (5) | 5 | - | - | - | 1 | 51.0±1.7 | 20 min 27 sec± 20 sec | 0.43±0.23 |
| Example 5 | P | PSS-1 (0.8) | 0.8 | - | - | - | 1 | 56.2±2.3 | 9 min 27 sec±23 sec | 1.06±0.20 |
| Example 6 | P | PSS-1 (5) | 5 | - | - | - | 1 | 50.6±1.8 | 20 min 43 sec±22 sec | 0.47±0.24 |
| Example 7 | P | PSS-1 (18) | 18 | - | - | - | 1 | 42.3±1.5 | 27min 05 sec±27 sec | 0.30±0.16 |
| Example 8 | L | PSS-2 (5) | 5 | - | - | - | 1 | 49.9±1.7 | 21 min 02 sec±25 sec | 0.48±0.28 |
| Example 9 | L | PSS-3 (5) | 5 | - | - | - | 1 | 47.3±1.5 | 21 min 37 sec±28 sec | 0.99±027 |
| Example 10 | L | PSS-4 (5) | 5 | - | - | - | 1 | 53.1±1.7 | 19 min 55 sec±23 sec | 0.44±0.21 |
| Example 11 | L | PSS-5 (5) | 5 | - | - | - | 1 | 53.8±1.6 | 19min 42 sec±24 sec | 0.42±0.20 |
| Example 12 | L | DBSS (5) | 5 | - | - | - | 1 | 51.9±1.3 | 18 min 23 sec±23 sec | 0.48±0.11 |
| Example 13 | L | PVSA (5) | 5 | - | - | - | 1 | 47.7±1.6 | 20 min 29 sec±26 sec | 1.04±0.30 |
| Example 14 | L | PSS-1 (2.5) | 5.7 | - | - | - | 0.7 | 53.6±1.8 | 7 min 19 sec± 29 sec | 0.29±0.10 |
| | | PVSA (3.2) | | | | | | | | |
| | | Na₂HPO₄ (0.8) | | | | | | | | |
| Example 15 | L | PSS-1 (2.5) | 5.7 | Silica | 0.016 | 10 | 1 | 61.2±1.7 | 5 min 11 sec± 10 sec | 0.11±0.05 |
| | | PVSA (3.2) | | | | | | | | |
| | | Na₂HPO₄ (0.8) | | | | | | | | |
| Comparative Example 1 | L | Na₂HPO₄ (0.8) | - | - | - | - | 1 | 59.7±2.7 | 8 min 58 sec±26 sec | 2.52±0.59 |
| Comparative Example 2 | L | PVA (5) | - | - | - | - | 1 | 29.9±1.4 | 35 min 43 sec±30 sec | 2.92±0.59 |
| | | Na₂HPO₄ (0.8) | | | | | | | | |
| Comparative Example 3 | L | ARG (5) | - | - | - | - | - | 35.3±1.5 | 32 min 37 sec±19 sec | Dye entered to cavity bottom |
| | | Na₂HPO₄ (0.8) | | | | | | | | |
| Comparative Example 4 | L | ACR (5) | - | - | - | - | 1 | 34.2±1.6 | 32 min 37 sec±19 sec | Dye entered to cavity bottom. |
| Comparative Example 5 | L | PSS-1(0.3) | 0.3 | - | - | - | 1 | 58.4±2.4 | 9 min 15 sec±22 sec | 2.24±0.39 |
| Comparative Example 6 | P | PSS-1 (22) | 22 | - | - | - | 1 | 29.1±1.3 | 52 min 46 sec*29 sec | 0.52±0.21 |

| | | | | | | | | | | |
|---|---|---|---|---|---|---|---|---|---|---|
| (*1) P: A powder agent contains a sulfonic acid salt and water is used as a liquid agent. L: An aqueous solution containing a sulfonic acid salt, a phosphoric acid salt, polyvinyl alcohol, an alginic acid salt, and/or a polyacrylic acid salt is used as a liquid agent. (*2) PSS-1: Sodium polystyrene sulfonate (molecular weight: about 1,000,000 Da) PSS-2: Sodium polystyrene sulfonate (molecular weight: about 500,000 Da) PSS-3: Sodium polystyrene sulfonate (molecular weight: about 50,000 Da) PSS-4: Sodium polystyrene sulfonate (molecular weight: about 2,260,000 Da) PSS-5: Sodium polystyrene sulfonate (molecular weight: about 5,640,000 Da) PVSA: Poly(sodium vinylsulfonate) DBSS: Sodium dodecylbenzenesulfonate PVA: Polyvinyl alcohol ARG: Sodium alginate ACR: Poly(sodium acrylate) (*3) TTCP: Tetracalcium phosphate particle DCPA: Anhydrous calcium hydrogen phosphate particle | | | | | | | | | | |

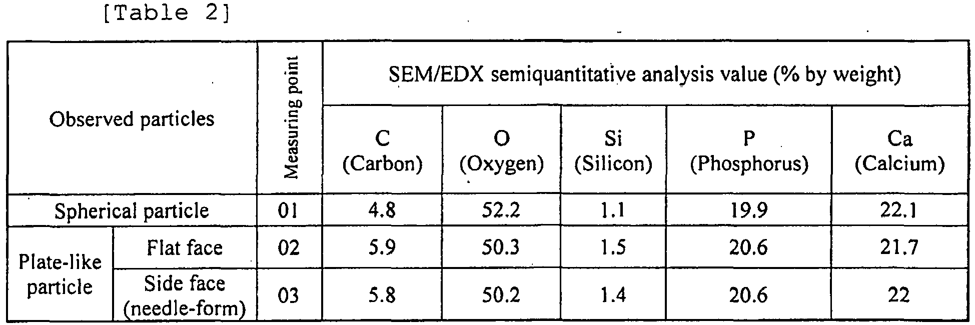

**[Table 3]**

| Observed particles | SEM excluding C (carbon)/EDX semiquantitative analysis value (% by weight) | | | | |
|---|---|---|---|---|---|
| | C (Carbon) | O (Oxygen) | Si (Silicon) | P (Phosphorus) | Ca (Calcium) |
| Large particle | - | 62.7 | 0.9 | 17.7 | 18.7 |
| Small particle A | - | 65.1 | 3.7 | 15.9 | 15.3 |
| Small particle B | - | 62.2 | 2.5 | 17.0 | 18.4 |

**[Table 4]**

| Observed particles | SEM including C (carbon)/EDX semiquantitative analysis value (% by weight) | | | | |
|---|---|---|---|---|---|
| | C (Carbon) | O (Oxygen) | Si (Silicon) | P (Phosphorus) | Ca (Calcium) |
| Large particle | 43.1 | 40.0 | 0.4 | 8.0 | 8.6 |
| Small particle A | 48.0 | 38.8 | 1.4 | 5.9 | 5.9 |
| Small particle B | 36.1 | 43.8 | 1.3 | 8.9 | 9.9 |

Table 1 shows that since Examples 1 to 11, and 13 to 15 in which 0.5 to 20 parts by weight of a polysulfonic acid salt (B) was contained based on 100 parts by weight of calcium phosphate particles (A) were shorter in marginal leakage distance than Comparative Example 1 in which no polysulfonic acid salt (B) was added and therefore the Examples were better in sealing ability of filled sites. This fact clearly establishes the effect produced by inclusion of a polysulfonic acid salt (B) in a certain amount.

Comparative Examples 2 to 4 in which an aqueous PVA solution, an aqueous ARG solution, and an aqueous ACR solution were used as a liquid agent instead of a polysulfonic acid salt (B) were lower in compressive strength, longer in setting time, and poorer in marginal sealing ability than Comparative Example 1 in which no polysulfonic acid salt (B) was added. Moreover, , Comparative Example 5 in which the content of a polysulfonic acid salt (B) was less than 0.5 part by weight was poorer in marginal sealing ability than Comparative Example 1 in which no polysulfonic acid salt (B) was added. Furthermore, in Comparative Example 6 in which the content of a polysulfonic acid salt (B) exceeded 20 parts by weight, the setting time was very long and the compressive strength lowered greatly.

Moreover, it was shown that the sealing ability is further improved by the fact that a calcium phosphate composition containing a certain amount of a polysulfonic acid salt (B) contains tetracalcium phosphate particles (A1), acidic calcium phosphate particles (A2), and inorganic particles (D) other than (A1) and (A2) and the acidic calcium phosphate particles (A2) contain acidic calcium phosphate composite particles covered with the inorganic particles (D) as in Example 15.

As shown by the results of the elemental analysis using an energy dispersive X-ray analyzer (EDX) of Table 2, the existing ratios of Si element at measuring points (+01, +02, and +03) of anhydrous calcium hydrogen phosphate composite particles surface were almost the same and anhydrous calcium hydrogen phosphate particles (A2) were found to be covered uniformly with inorganic particles (D). On the other hand, as shown by the results of the elemental analysis of Tables 3 and 4, in Referential Example in which anhydrous calcium hydrogen phosphate composite particles were not formed well, the existing ratio of Si element of small particles was higher than that of Si element on the surface of large particles and therefore the existing ratio of silica particles on the surface of anhydrous calcium hydrogen phosphate particles (A2) were uneven.

## Claims

1. A process for producing a calcium phosphate composition powder comprising calcium phosphate particles (A) and a polysulfonic acid salt (B),
wherein the calcium phosphate composition powder contains 0.5 to 20 parts by weight of the polysulfonic acid salt (B) based on 100 parts by weight of the calcium phosphate particles (A), wherein the calcium phosphate particles (A) consist of tetracalcium phosphate particles (A1) and acidic calcium phosphate particles (A2), and the tetracalcium phosphate particles (A1), the acidic calcium phosphate particles (A2) and the polysulfonic acid salt (B) are mixed in a state of a powder.

2. The process for producing a calcium phosphate composition powder according to claim 1, wherein the polysulfonic acid salt (B) is a polystyrenesulfonic acid salt and/or a polyvinylsulfonic acid salt.

3. The process for producing a calcium phosphate composition powder according to claim 1 or 2, wherein the calcium phosphate composition powder further comprises an alkali metal salt of phosphoric acid (C).

4. The process for producing a calcium phosphate composition powder according to claim 3, wherein the alkali metal salt of phosphoric acid (C) is disodium hydrogen phosphate and/or sodium dihydrogen phosphate.

5. The process for producing a calcium phosphate composition powder according to any one of claims 1 to 4, wherein a blending ratio (A1/A2) of the tetracalcium phosphate particles (A1) to the acidic calcium phosphate particles (A2) is from 40/60 to 60/40 in molar ratio.

6. The process for producing a calcium phosphate composition powder according to any one of claims 1 to 5, wherein the composition contains acidic calcium phosphate composite particles which contain 1 to 30 parts by weight of inorganic particles (D) other than (A1) and (A2) based on 100 parts by weight of the acidic calcium phosphate particles (A2) and in which the acidic calcium phosphate particles (A2) are covered with the inorganic particles (D).

7. The process for producing a calcium phosphate composition powder according to any one of claims 1 to 6, wherein the acidic calcium phosphate particles (A2) and inorganic particles (D) other than (A1) and (A2) are mechanochemically hybridized and then mixed with the tetracalcium phosphate particles (A1) and the polysulfonic acid salt (B) in a state of a powder.

8. A process for producing a calcium phosphate composition paste comprising calcium phosphate particles (A) and a polysulfonic acid salt (B),
wherein a liquid comprising water as a main component is added to a powder of a calcium phosphate composition comprising 0.5 to 20 parts by weight of the polysulfonic acid salt (B) based on 100 parts by weight of the calcium phosphate particles (A), followed by kneading, wherein the calcium phosphate particles (A) consist of tetracalcium phosphate particles (A1) and acidic calcium phosphate particles (A2), and the tetracalcium phosphate particles (A1), the acidic calcium phosphate particles (A2) and the polysulfonic acid salt (B) are mixed in a state of a powder.

9. A process for producing a calcium phosphate composition paste comprising calcium phosphate particles (A) and a polysulfonic acid salt (B), wherein an aqueous solution containing 0.5 to 20 parts by weight of the polysulfonic acid salt (B) based on 100 parts by weight of the calcium phosphate particles (A) is added to a powder comprising the calcium phosphate particles (A), followed by kneading, wherein the calcium phosphate particles (A) consist of tetracalcium phosphate particles (A1) and acidic calcium phosphate particles (A2).

## Patentansprüche

1. Verfahren zur Herstellung eines Calciumphosphatzusammensetzungspulvers, umfassend Calciumphosphatteilchen (A) und ein Polysulfonsäuresalz (B), wobei das Calciumphosphatzusammensetzungspulver 0,5 bis 20 Gew.-Teile des Polysulfonsäuresalzes (B), basierend auf 100 Gew.-Teilen der Calciumphosphatteilchen (A) enthält, wobei die Calciumphosphatteilchen (A) aus Tetracalciumphosphatteilchen (A1) und sauren Calciumphosphatteilchen (A2) bestehen, und die Tetracalciumphosphatteilchen (A1), die sauren Calciumphosphatteilchen (A2) und das Polysulfonsäuresalz (B) in einem Zustand eines Pulvers gemischt sind.

2. Verfahren zur Herstellung eines Calciumphosphatzusammensetzungspulvers gemäß Anspruch 1, wobei das Polysulfonsäuresalz (B) ein Polystyrolsulfonsäuresalz und/oder ein Polyvinylsulfonsäuresalz ist.

3. Verfahren zur Herstellung eines Calciumphosphatzusammensetzungspulvers gemäß Anspruch 1 oder 2, wobei das Calciumphosphatzusammensetzungspulver weiter ein Alkalimetallsalz von Phosphorsäure (C) umfaßt.

4. Verfahren zur Herstellung eines Calciumphosphatzusammensetzungspulvers gemäß Anspruch 3, wobei das Alkalimetallsalz von Phosphorsäure (C) Dinatriumhydrogenphosphat und/oder Natriumhydrogenphosphat ist.

5. Verfahren zur Herstellung eines Calciumphosphatzusammensetzungspulvers gemäß einem der Ansprüche 1 bis 4, wobei ein Mischungsverhältnis (A1/A2) der Tetracalciumphosphatteilchen (A1) zu den sauren Calciumphosphatteilchen (A2) von 40/60 bis 60/40 im Molverhältnis ist.

6. Verfahren zur Herstellung eines Calciumphosphatzusammensetzungspulvers gemäß einem der Ansprüche 1 bis 5, wobei die Zusammensetzung saure Calciumphosphatkompositteilchen enthält, welche 1 bis 30 Gew.-Teile anorganischer Teilchen (D), die anders als (A1) und (A2) sind, bezogen auf 100 Gew.-Teile der sauren Calciumphosphatteilchen (A2), enthalten und worin die sauren Calciumphosphatteilchen (A2) mit den anorganischen Teilchen (D) bedeckt sind.

7. Verfahren zur Herstellung eines Calciumphosphatzusammensetzungspulvers gemäß einem der Ansprüche 1 bis 6, wobei die sauren Calciumphosphatteilchen (A2) und anorganischen Teilchen (D), die anders als (A1) und (A2) sind, mechanisch-chemisch hybridisiert werden und anschließend mit den Tetracalciumphosphatteilchen (A1) und dem Polysulfonsäuresalz (B) in einem Zustand eines Pulvers gemischt werden.

8. Verfahren zur Herstellung einer Calciumphosphatzusammensetzungspaste, umfassend Calciumphosphatteilchen (A) und ein Polysulfonsäuresalz (B), wobei eine Flüssigkeit, umfassend Wasser als eine Hauptkomponente, zu einem Pulver einer Calciumphosphatzusammensetzung, umfassend 0,5 bis 20 Gew.-Teile des Polysulfonsäuresalzes (B), bezogen auf 100 Gew.-Teile der Calciumphosphatteilchen (A), gegeben wird, gefolgt von Kneten, wobei die Calciumphophatteilchen (A) aus Tetracalciumphosphatteilchen (A1) und sauren Calciumphosphatteilchen (A2) bestehen, und die Tetracalciumphosphatteilchen (A1), die sauren Calciumphosphatteilchen (A2) und das Polysulfonsäuresalz (B) in einem Zustand eines Pulvers gemischt werden.

9. Verfahren zur Herstellung einer Calciumphosphatzusammensetzungspaste, umfassend Calciumphosphatteilchen (A) und ein Polysulfonsäuresalz (B), wobei eine wässrige Lösung, enthaltend 0,5 bis 20 Gew.-Teile des Polysulfonsäuresalzes (B), bezogen auf 100 Gew.-Teile der Calciumphosphatteilchen (A), zu einem Pulver, umfassend die Calciumphosphatteilchen (A), gegeben wird, gefolgt von Kneten, wobei die Calciumphosphatteilchen (A) aus Tetracalciumphosphatteilchen (A1) und sauren Calciumphosphatteilchen (A2) bestehen.

## Revendications

1. Procédé de production d'une poudre de composition à base de phosphate de calcium comprenant des particules de phosphate de calcium (A) et un sel d'acide polysulfonique (B),
dans lequel la poudre de composition à base de phosphate de calcium contient de 0,5 à 20 parties en poids du sel d'acide polysulfonique (B) sur la base de 100 parties en poids des particules de phosphate de calcium (A), dans lequel les particules de phosphate de calcium (A) consistent en des particules de phosphate de tétracalcium (A1) et des particules de phosphate de calcium acide (A2), et les particules de phosphate de tétracalcium (A1), les particules de phosphate de calcium acide (A2) et le sel d'acide polysulfonique (B) sont mélangés à l'état de poudre.

2. Procédé de production d'une poudre de composition à base de phosphate de calcium selon la revendication 1, dans lequel le sel d'acide polysulfonique (B) est un sel d'acide polystyrènesulfonique et/ou un sel d'acide polyvinylsulfonique.

3. Procédé de production d'une poudre de composition à base de phosphate de calcium selon la revendication 1 ou 2, dans lequel la poudre de composition à base de phosphate de calcium comprend en outre un sel de métal alcalin d'acide phosphorique (C).

4. Procédé de production d'une poudre de composition à base de phosphate de calcium selon la revendication 3, dans lequel le sel de métal alcalin d'acide phosphorique (C) est du phosphate d'hydrogène disodique et/ou du phosphate dihydrogène de sodium.

5. Procédé de production d'une poudre de composition à base de phosphate de calcium selon l'une quelconque des revendications 1 à 4, dans lequel un ratio de mélange (A1/A2) des particules de phosphate de tétracalcium (A1) avec les particules de phosphate de calcium acide (A2) présente un rapport molaire dans la plage de 40/60 à 60/40.

6. Procédé de production d'une poudre de composition à base de phosphate de calcium selon l'une quelconque des revendications 1 à 5, dans lequel la composition contient des particules de composite de phosphate de calcium acide qui contiennent de 1 à 30 parties en poids de particules inorganiques (D) autres que (A1) et (A2) sur la base de 100 parties en poids des particules de phosphate de calcium acide (A2) et dans lequel les particules de phosphate de calcium acide (A2) sont recouvertes par les particules inorganiques (D).

7. Procédé de production d'une poudre de composition à base de phosphate de calcium selon l'une quelconque des revendications 1 à 6, dans lequel les particules de phosphate de calcium acide (A2) et les particules inorganiques (D) autre que (A1) et (A2) sont soumises à une hybridation mécanochimique et ensuite mélangées avec les particules de phosphate de tétracalcium (A1) et le sel d'acide polysulfonique (B) dans un état de poudre.

8. Procédé de production d'une pâte de composition à base de phosphate de calcium comprenant des particules de phosphate de calcium (A) et un sel d'acide polysulfonique (B),
dans lequel un liquide comprenant de l'eau en tant que composant principal est ajouté à la poudre d'une composition à base de phosphate de calcium comprenant de 0,5 à 20 parties en poids du sel d'acide polysulfonique (B) sur la base de 100 parties en poids des particules de phosphate de calcium (A), avec ensuite un malaxage, dans lequel les particules de phosphate de calcium (A) consistent en des particules de phosphate de tétracalcium (A1) et des particules de phosphate de calcium acide (A2), et les particules de phosphate de tétracalcium (A1), les particules de phosphate de calcium acide (A2) et le sel d'acide polysulfonique (B) sont mélangées à l'état de poudre.

9. Procédé de production d'une pâte de composition à base de phosphate de calcium comprenant des particules de phosphate de calcium (A) et un sel d'acide polysulfonique (B), dans lequel une solution aqueuse contenant de 0,5 à 20 parties en poids du sel d'acide polysulfonique (B) sur la base de 100 parties en poids des particules de phosphate de calcium (A) est ajoutée à une poudre comprenant les particules de phosphate de calcium (A), avec ensuite un malaxage, dans lequel les particules de phosphate de calcium (A) consistent en des particules de phosphate de tétracalcium (A1) et des particules de phosphate de calcium acide (A2).
